# EUROPEAN PATENT APPLICATION

(11) **EP 4 005 614 A1**
(43) Date of publication of application: **01.06.2022**
(21) Application number: 20843984.4
(22) Date of filing: 25.06.2020
(51) Int. Cl.: A61M 5/14, A61M 5/145

(54) **DRUG SOLUTION INJECTION DEVICE**

(30) Priority: 25.07.2019 JP 2019136780
(71) Applicant: Asahi Intecc Co., Ltd., Seto-shi, Aichi 489-0071 (JP)
(72) Inventor: NAMIMA, Satoshi, Seto-shi, Aichi 489-0071 (JP)
(74) Representative: Winter, Brandl - Partnerschaft mbB
(86) International application number: PCT/JP2020/024993
(87) International publication number: WO 2021/014877

(57) **Abstract**

A drug solution injection device includes: a catheter that has a hollow shaft, a hollow needle portion disposed on a distal end of the hollow shaft, and a lumen formed inside each of the hollow shaft and the needle portion and containing a drug solution; and a wire that is inserted into the lumen and is advanced from a proximal end of the lumen toward a distal end side so as to discharge the drug solution in the lumen from a distal end of the needle portion.

## Description

### TECHNICAL FIELD

The disclosed embodiments relate to a drug solution injection device.

### BACKGROUND ART

There are known devices for injecting a drug solution into a patient's body using a catheter that can be inserted into a living body lumen. For example, Patent Document 1 discloses an injection device including an injection catheter and a hand pump connected to a proximal end of the injection catheter. For example, Patent Document 2 discloses a drug solution injection device including an extension tube and a syringe connected to a proximal end of the extension tube, where an RFID (Radio Frequency Identification) chip storing various recorded data is attached to the syringe.

### CITATION LIST

### Patent Literature

Patent Literature 1: JP 2001-519212 W
Patent Literature 2: WO2005/084732

### SUMMARY OF INVENTION

There are various doses for drug solutions that are injected into a patient's body. In this regard, the technologies described in Patent Literature 1 and Patent Literature 2 have had a problem that it is impossible to respond to a request for injecting a trace amount of the drug solution into a patient's body because an injection dose of the drug solution cannot be precisely controlled. Also, the technologies described in Patent Literature 1 and Patent Literature 2 have had a problem that the drug solution is wasted because the drug solution remains in the injection catheter and the extension tube after use. Furthermore, the technology described in Patent Literature 2 has had a problem that the drug solution injection device is large and cannot be not easily installed and set up.

The disclosed embodiments were made to solve at least a part of the aforementioned problems, and an object of the disclosed embodiments is to provide a drug solution injection device capable of precisely controlling an injection dose of a drug solution and reducing waste of the drug solution.

The disclosed embodiments were made to solve at least a part of the aforementioned problems, and can be achieved as the following aspects.
(1) According to one aspect of the disclosed embodiments, a drug solution injection device is provided. This drug solution injection device includes: a catheter that has a hollow shaft, a hollow needle portion disposed on a distal end of the hollow shaft, and a lumen formed inside each of the hollow shaft and the needle portion and containing a drug solution; and a wire that is inserted into the lumen and is advanced from a proximal end of the lumen toward a distal end side so as to discharge the drug solution in the lumen from a distal end of the needle portion.
   According to this configuration, the drug solution injection device includes a wire to be inserted into a lumen for containing a drug solution and advanced from a proximal end of the lumen toward a distal end side so as to discharge the drug solution in the lumen from a distal end of the needle portion. That means, in this configuration, the drug solution in the lumen is extruded by the wire so as to be discharged from the distal end of the needle portion. Thereby, an amount of the drug solution to be discharged can be the same as a volume of the wire, and therefore the injection dose of the drug solution can be precisely controlled. In this configuration, the drug solution in the lumen can be thoroughly used by extruding the drug solution from the lumen by the wire. Thus, waste of the drug solution can be reduced.
(2) In the drug solution injection device according to the aforementioned aspect, an outer diameter of the wire may be substantially constant from the distal end to the proximal end.
   According to this configuration, since the outer diameter of the wire is substantially constant from the distal end to the proximal end, an amount of the drug solution to be discharged from the distal end of the needle portion can be easily comprehended depending on the length of the wire advanced in the lumen.
(3) The drug solution injection device according to the aforementioned aspect may further include an accommodation portion that is connected to the proximal end portion of the catheter, accommodates a part of the wire on the proximal end side, and allows the wire to advance and retract in the lumen.
   According to this configuration, the drug solution injection device includes an accommodation portion connected to the proximal end portion of the catheter, accommodating a part of the wire on the proximal end side, and allowing the wire to advance and retract in the lumen. Thereby, the wire can be advanced and retracted without direct contact between an operator and the wire.
(4) In the drug solution injection device according to the aforementioned aspect, the accommodation portion may advance the wire in the lumen, so that the drug solution in an amount corresponding to a length of the advanced wire is discharged from the distal end of the needle portion.
   According to this configuration, the accommodation portion allows the drug solution in an amount corresponding to the length of the advanced wire to be discharged from the distal end of the needle portion. Thereby, the injection dose of the drug solution can be precisely controlled and waste of the drug solution can be reduced.
(5) In the drug solution injection device according to the aforementioned aspect, the accommodation portion may feed the wire toward the lumen to advance the wire, and wind the wire from the lumen to retract the wire.
   According to this configuration, since the accommodation portion feeds the wire to the lumen to advance the wire and winds the wire from the lumen to retract the wire, the wire can be reliably advanced and retracted. In addition, since the wire is wound for accommodation, the accommodation portion can be downsized.
(6) In the drug solution injection device according to the aforementioned aspect, the accommodation portion includes a wire winding portion around which a part of the wire on the proximal end side is wound, and a handle portion connected to the wire winding portion. The wire winding portion may feed and wind the wire in a length corresponding to a rotation quantity of the handle portion.
   According to this configuration, the accommodation portion includes the handle portion connected to the wire winding portion, and the wire winding portion feeds and winds the wire in a length corresponding to a rotation quantity of the handle portion. Thereby, an operator can easily feed and wind the wire by rotating the handle portion.
(7) In the drug solution injection device according to the aforementioned aspect, the accommodation portion further includes a wire extruding portion that extrudes the wire fed from the wire winding portion, toward the lumen. The wire extruding portion includes first and second rollers that rotate in response to the rotation of the handle portion, and may extrude the wire while the wire is sandwiched between the first and second rollers.
   According to this configuration, since the accommodation portion includes the wire extruding portion that extrudes the wire fed from the wire winding portion toward the lumen, the wire can be reliably pushed and advanced into the lumen.
(8) In the drug solution injection device according to the aforementioned aspect, in response to the same rotation quantity of the handle portion, the length of the wire to be extruded by the wire extruding portion may be longer than the length of the wire to be fed by the wire winding portion.
   According to this configuration, in response to the same rotation quantity of the handle portion, the length of the wire to be extruded by the wire extruding portion is longer than the length of the wire to be fed by the wire winding portion, and therefore the wire can be reliably pushed and advanced into the lumen.
(9) In the drug solution injection device according to the aforementioned aspect, the accommodation portion may further include a valve member that is disposed between the wire extruding portion and the proximal end portion of the catheter to restrict the drug solution in the lumen from moving into the accommodation portion.
   According to this configuration, the accommodation portion includes the valve member for restricting the drug solution in the lumen from moving into the accommodation portion. Thereby, the drug solution in the lumen can be prevented from entering the accommodation portion, and the inside of the accommodation portion can be maintained in an airtight state.
(10) In the drug solution injection device according to the aforementioned aspect, the accommodation portion may allow the drug solution in an amount corresponding to the rotation quantity of the handle portion to be discharged from the distal end of the needle portion.
   According to this configuration, the accommodation portion allows the drug solution in an amount corresponding to the rotation quantity of the handle portion to be discharged from the distal end of the needle portion. Thereby, the amount of the drug solution to be discharged from the distal end of the needle portion can be easily controlled depending on the rotation quantity of the handle portion. As a result, the injection dose of the drug solution can be precisely controlled and waste of the drug solution can be reduced.
(11) In the drug solution injection device according to the aforementioned aspect, the catheter further includes a connector having a drug solution feed port for feeding the drug solution to the lumen and a wire insertion port for inserting the wire into the lumen, connected to the proximal end portion of the hollow shaft, and in communication with the lumen. The drug solution feed port may be disposed on the distal end side with respect to the wire insertion port.
   According to this configuration, since the catheter further includes a connector, convenience of inserting the wire into the catheter can be improved. The connector is configured such that the drug solution feed port is disposed on the distal end side with respect to the wire insertion port. Thus, the drug solution can be fed from the drug solution feed port without inhibition of the feed due to the wire, while the distal end portion of the wire is inserted into the wire insertion port.
(12) In the drug solution injection device according to the aforementioned aspect, the catheter further includes the connector having the drug solution feed port for feeding the drug solution to the lumen and the wire insertion port for inserting the wire into the lumen, connected to the proximal end portion of the hollow shaft, and in communication with the lumen. The drug solution feed port may be disposed on the distal end side with respect to the distal end of the wire maximally wound around the wire winding portion by the rotation of the handle portion.
   According to this configuration, the drug solution feed port of the connector is disposed on the distal end side with respect to the distal end of the wire maximally wound around the wire winding portion. Thus, the drug solution can be fed from the drug solution feed port without inhibition of the feed due to the wire when the wire is maximally wound around the accommodation portion by rotating the handle portion.
(13) In the drug solution injection device according to the aforementioned aspect, the connector further includes a first extension portion extending in a longitudinal direction of the catheter and a second extension portion extending from a lateral side of the first extension portion on the distal end side of the first extension portion. The drug solution feed port may be formed on a distal end portion of the second extension portion, and the wire insertion port may be formed on a proximal end portion of the first extension portion.
   According to this configuration, the connector is configured such that the wire insertion port is formed on the proximal end portion of the first extension portion, and the drug solution feed port is formed on the distal end portion of the second extension portion extending from the lateral side of the first extension portion on the distal end side of the first extension portion. Thereby, insertion of the wire into the wire insertion port and feed of the drug solution from the drug solution feed port can be smoothly carried out.
(14) In the drug solution injection device according to the aforementioned aspect, each transverse sectional shape of the lumen and the wire is almost circular, and the outer diameter of the wire may be 80% or larger of an inner diameter of the lumen.
   According to this configuration, each transverse sectional shape of the lumen and the wire is almost circular, and the outer diameter of the wire is 80% or larger of the inner diameter of the lumen. Thereby, the drug solution in the lumen can be extruded by the wire while maintaining slidability of the wire in the lumen.

The disclosed embodiments can be achieved in various aspects e.g. in a configuration of a drug solution injection device, a catheter and wire for injecting the drug solution, a wire and accommodation portion for injecting the drug solution, a system including a drug solution injection device, a system including a wire and accommodation portion, a method for manufacturing these devices and systems, and the like.

### BRIEF DESCRIPTION OF DRAWINGS

Fig. 1 is an explanatory diagram illustrating a configuration of a drug solution injection device according to the first embodiment.
Fig. 2 is an explanatory diagram illustrating a sectional configuration of a catheter.
Fig. 3 is an explanatory diagram illustrating a configuration of an accommodation portion.
Fig. 4 is an explanatory diagram illustrating a configuration of a wire winding portion, a gear, and a wire extruding portion.
Fig. 5 is an explanatory diagram illustrating a configuration of a valve member.
Fig. 6 is an explanatory diagram illustrating a configuration of a wire.
Fig. 7 is a diagram for explaining advancement of the wire and discharge of a drug solution.
Fig. 8 is a diagram for explaining retraction of the wire.
Fig. 9 is an explanatory diagram illustrating a configuration of an accommodation portion according to the second embodiment.
Fig. 10 is an explanatory diagram illustrating a configuration of an accommodation portion according to the third embodiment.
Fig. 11 is an explanatory diagram illustrating a configuration of an accommodation portion according to the fourth embodiment.
Fig. 12 is an explanatory diagram illustrating a configuration of a wire according to the fifth embodiment.
Fig. 13 is an explanatory diagram illustrating a configuration of a wire according to the sixth embodiment.
Fig. 14 is an explanatory diagram illustrating a configuration of a drug solution injection device according to the seventh embodiment.
Fig. 15 is an explanatory diagram illustrating a configuration of a drug solution injection device according to the eighth embodiment.

### EMBODIMENTS OF THE INVENTION

### <First Embodiment>

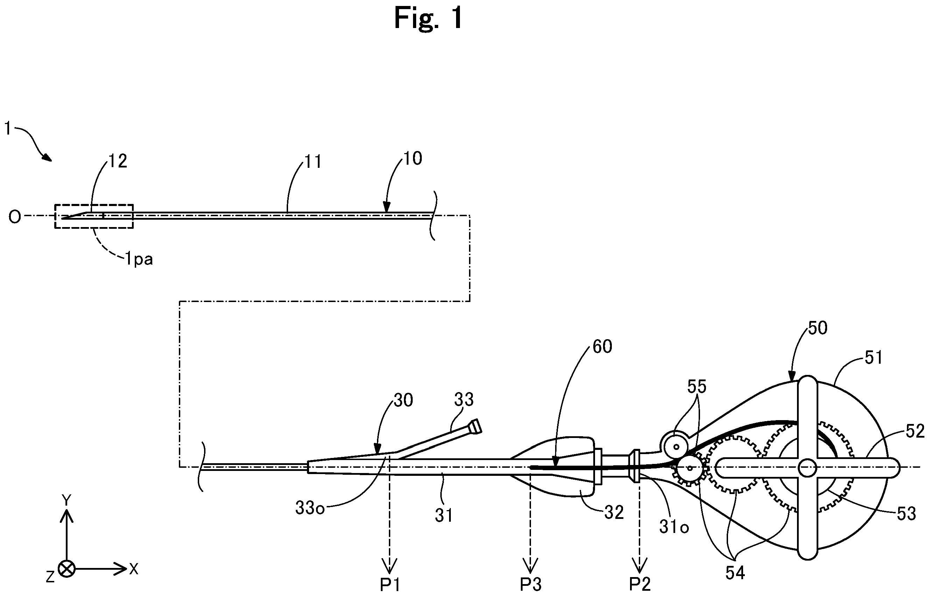

Fig. 1 is an explanatory diagram illustrating a configuration of a drug solution injection device 1 according to the first embodiment. The drug solution injection device 1 is used for injecting a drug solution into a patient's body using a catheter that can be inserted into a living body lumen. Herein, the living body lumen includes various lumens such as a vascular system, a lymphatic gland system, a biliary system, a urinary tract system, a respiratory system, a digestive system, a secretory gland and a reproductive organ. The drug solution injection device 1 includes a catheter 10, an accommodation portion 50, and a wire 60.

In Fig. 1, an axis passing through the center of the drug solution injection device 1 is represented by an axis line O (dot and dash line). In the example of Fig. 1, the axis line O is consistent with an axis passing through each center of the catheter 10 and the accommodation portion 50. However, the axis line O may be inconsistent with each central axis of the catheter 10 and the accommodation portion 50. In Fig. 1, XYZ axes that are orthogonal to each other are illustrated. The X axis corresponds to a length direction of the drug solution injection device 1, the Y axis corresponds to a width direction of the drug solution injection device 1, and the Z axis corresponds to a height direction of the drug solution injection device 1. The left side (-X axis direction) in Fig. 1 is referred to as a "distal end side" of the drug solution injection device 1 and each component, and the right side in Fig. 1 (+X axis direction) is referred to as a "proximal end side" of the drug solution injection device 1 and each component. As for the drug solution injection device 1 and each component, an end portion positioned on the distal end side is referred to as a "distal end", and the distal end and the vicinity thereof are referred to as a "distal end portion". In addition, an end portion positioned on the proximal end side is referred to as a "proximal end", and the proximal end and the vicinity thereof are referred to as a "proximal end portion". The distal end side is inserted into a living body, and the proximal end side is operated by an operator such as a surgeon. These definitions are common for the figures following Fig. 1.

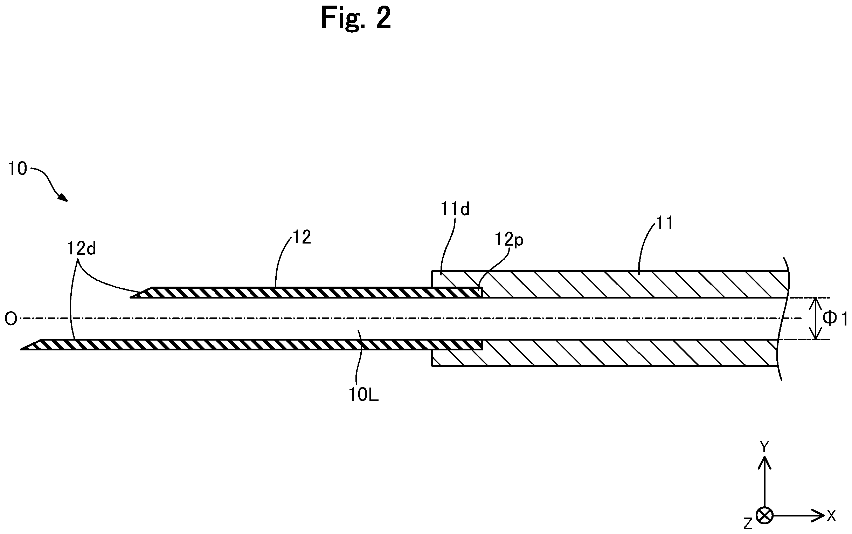

Fig. 2 is an explanatory diagram illustrating a sectional configuration of the catheter 10. Fig. 2 presents a sectional configuration of a part 1pa (Fig. 1: dashed frame) of the catheter 10 on the distal end side. The XYZ axes in Fig. 2 correspond to the XYZ axes in Fig. 1. The catheter 10 is inserted into a living body lumen of a patient and is used to transport a drug solution to a desired position in the patient's body to inject the drug solution into the desired position in the patient's body. The catheter 10 includes a hollow shaft 11, a needle portion 12, and a connector 30.

The hollow shaft 11 is a long member extending along the axis line O. The hollow shaft 11 is a hollow and almost cylindrical member, in which an opening is formed on each of the distal end portion and the proximal end portion, and an inner cavity communicating between the both openings is formed thereinside. A coil body or a braided body may be embedded in a thick-walled portion of the hollow shaft 11 to improve at least some of flexibility, torquability, pushability, kink resistance, blood vessel followability, lesion passableness, and guidewire operability. The hollow shaft 11 may be composed of multiple layers made of the same or different materials.

The needle portion 12 is a hollow needle in which an opening is formed on each of a distal end portion 12d and a proximal end portion 12p, and an inner cavity communicating between the both openings is formed thereinside (Fig. 2). In the needle portion 12, a sharp needle tip is formed on the distal end portion 12d, and the proximal end portion 12p is joined to a distal end portion 11d of the hollow shaft 11. The joining of the needle portion 12 can be carried out using any joining agent such as an epoxy adhesive. Herein, the needle portion 12 is joined to the hollow shaft 11 while the inner cavity of the needle portion 12 and the inner cavity of the hollow shaft 11 are in communication with each other. Both the inner cavity of the needle portion 12 and the inner cavity of the hollow shaft 11 have a substantially constant inner diameter Φ1. The inner cavity of the needle portion 12 and the inner cavity of the hollow shaft 11 constitute a lumen 10L that contains the drug solution. The transverse sectional shape of the lumen 10L is almost circular.

The connector 30 is connected to the proximal end portion of the hollow shaft 11 and is used for feeding the drug solution to the catheter 10 and inserting the wire into the catheter 10. The connector 30 includes a first extension portion 31, a blade portion 32, and a second extension portion 33.

The first extension portion 31 is an almost cylindrical member connected to the proximal end portion of the hollow shaft 11 and extending along the axis line O. An inner cavity extending along the axis line O is formed inside the first extension portion 31. In the example of Fig. 1, in addition to the inner cavity of the needle portion 12 and the inner cavity of the hollow shaft 11 described above, the inner cavity of the first extension portion 31 also constitutes the lumen 10L. A wire insertion port 31o communicating between the lumen 10L and the outside is formed on the proximal end portion of the first extension portion 31. The second extension portion 33 is an almost cylindrical member extending from a lateral side of the first extension portion 31 in a direction different from the first extension portion 34 on the distal end side of the first extension portion 31. An inner cavity in communication with the lumen 10L is formed inside the second extension portion 33. A drug solution feed port 33o communicating between the lumen 10L and the inner cavity of the second extension portion 33 is formed on the distal end portion of the second extension portion 33.

As illustrated in Fig. 1, in the catheter 10 according to the first embodiment, a position P1 of the drug solution feed port 33o is positioned on the distal end side with respect to a position P2 of the wire insertion port 31o. In addition, the position P1 of the drug solution feed port 33o is positioned on the distal end side with respect to a position P3 on the distal end of the wire 60 maximally wound around a wire winding portion 53 of the accommodation portion 50 described later. The blade portion 32 is composed of two blade-shaped members extending from the lateral side of the first extension portion 34 in the ±Y axis direction on the proximal end side of the first extension portion 31. The blade portion 32 is used when the operator grasps the connector 30. At least some parts of the first extension portion 31, the blade portion 32, and the second extension portion 33, constituting the connector 30 may be integrally configured.

Preferably, the hollow shaft 11 has antithrombogenicity, flexibility, and biocompatibility, and can be made of a resin material or a metal material. As the resin material, for example, a polyamide resin, a polyolefin resin, a polyester resin, a polyurethane resin, a silicon resin, a fluororesin, or the like can be adopted. As the metal material, for example, a stainless steel such as SUS304, an NiTi alloy, a cobalt-chromium alloy, or the like can be used. Preferably, the needle portion 12 has antithrombogenicity and biocompatibility, and can be made of a metal material of a stainless steel such as SUS304, an NiTi alloy, a cobalt-chromium alloy, or the like. The connector 30 can be made of a resin material, e.g. a polyurethane, a polypropylene, a hard polyvinyl chloride, or the like.

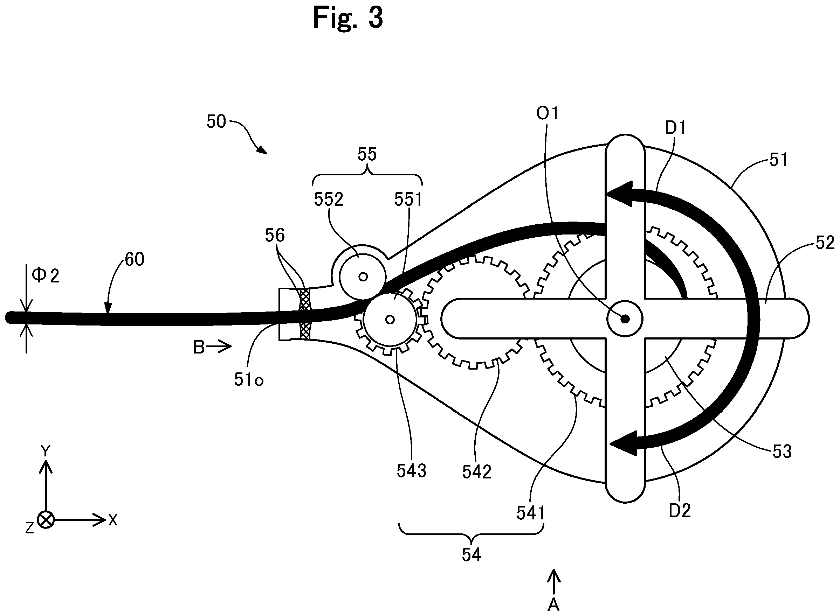

Fig. 3 is an explanatory diagram illustrating a configuration of the accommodation portion 50. The accommodation portion 50 accommodates a part of the wire 60 on the proximal end side and is used to achieve advancement of the wire 60 toward the lumen 10L of the catheter 10, and retraction of the wire 60 from the lumen 10L. The accommodation portion 50 includes a housing 51, a handle portion 52, the wire winding portion 53, a gear 54, a wire extruding portion 55, and a valve member 56.

The housing 51 refers to a case that accommodates the wire winding portion 53, the gear 54, the wire extruding portion 55, and the valve member 56 thereinside (inner part). In Fig. 1 and Fig. 3, for convenience of illustration, the housing 51 is represented only by its outline as a transparent member, and each member accommodated in the housing 51 is represented by solid lines. The handle portion 52 refers to a handle disposed outside the housing 51 and capable of rotating in a first direction D1 and a second direction D2 represented by arrows around a rotation shaft O1. The rotation shaft O1 of the handle portion 52 extends inside the housing 51 and is connected to the gear 54 (first gear 541 described later). A rotative force applied to the handle portion 52 is transmitted to the gear 54 by the rotation shaft O1.

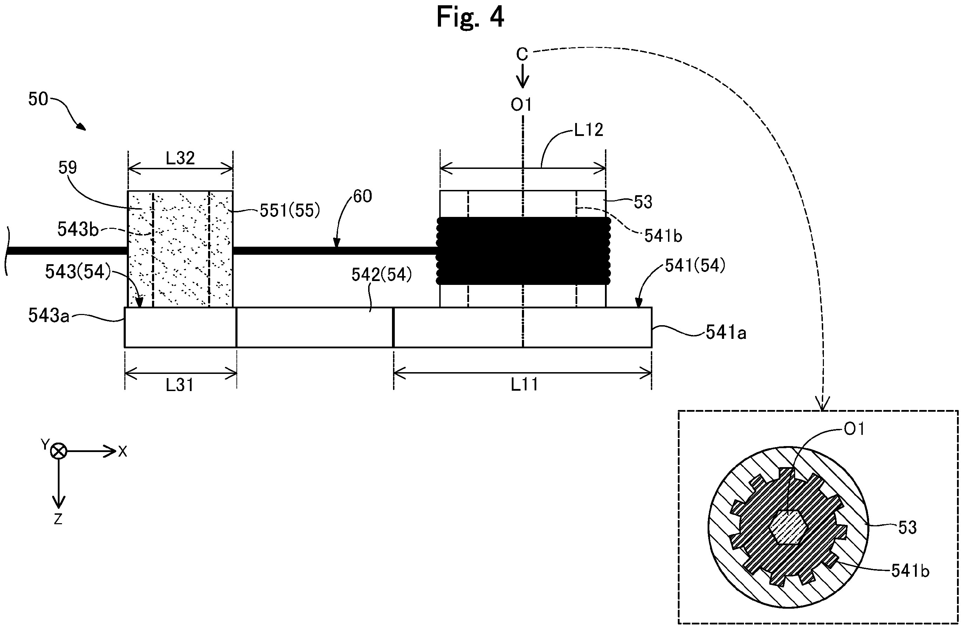

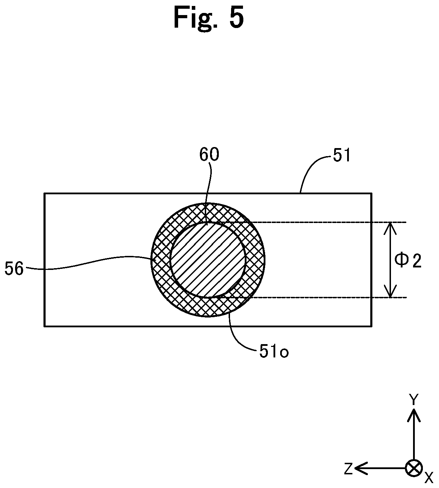

Fig. 4 is an explanatory diagram illustrating a configuration of the wire winding portion 53, the gear 54, and the wire extruding portion 55. In the upper part of Fig. 4, the wire winding portion 53, the gear 54, the wire extruding portion 55, and the wire 60 viewed from direction A in Fig. 3 are illustrated. The lower part of Fig. 4 (in the dashed frame), the rotation shaft O1, the wire winding portion 53, and the first gear 541 viewed from direction C in Fig. 4 are illustrated. Fig. 5 is an explanatory diagram illustrating a configuration of the valve member 56. In Fig. 5, the housing 51, the valve member 56, and the wire 60 viewed from direction B in Fig. 3 are illustrated. The XYZ axes in Fig. 4 and Fig. 5 correspond to the XYZ axes in Fig. 1 respectively.

The gear 54 includes the first gear 541, a second gear 542, and a third gear 543. As illustrated in Fig. 4, the first gear 541 is convex-shaped and has outer teeth 541a and inner teeth 541b. Similarly, the third gear 543 is convex-shaped and has outer teeth 543a and inner teeth 543b. The first gear 541 is connected to the handle portion 52 via the rotation shaft O1. The second gear 542 engages with the outer teeth 541a of the first gear 541 and the outer teeth 543a of the third gear 543 such that a rotative force transmitted from the handle portion 52 to the first gear 541 is transmitted to the third gear 543. A reference circle diameter L11 of the outer teeth 541a of the first gear 541 is larger than a reference circle diameter L31 of the outer teeth 543a of the third gear 543 (Fig. 4). In other words, the number of the outer teeth 541a of the first gear 541 is more than the number of the outer teeth 543a of the third gear 543. In the first embodiment, the reference circle diameter of the gear means an average of a circle diameter of a tooth top and a circle diameter of a tooth bottom.

The wire winding portion 53 is an almost cylindrical member fitted into the inner teeth 541b of the first gear 541 and rotates in response to the rotation of the first gear 541 (lower part of Fig. 4). An outer diameter of the wire winding portion 53 is represented by L12 (Fig. 4). Apart of the wire 60 on the proximal end side is previously wound around the wire winding portion 53. The proximal end portion of the wire 60 may or may not be fixed to the wire winding portion 53. The wire winding portion 53 and the first gear 541 may be integrally formed.

The wire extruding portion 55 includes a first roller 551 and a second roller 552. The first roller 551 is an almost cylindrical member fitted into the inner teeth 543b of the third gear 543 and rotates in response to the rotation of the third gear 543. An outer diameter of the first roller 551 is represented by L32 (Fig. 4). A lateral side of the first roller 551 is covered by a covering portion 59. The first roller 551 and the third gear 543 may be integrally formed. The second roller 552 is an almost cylindrical or almost columnar member disposed adjacent to the first roller 551 and rotates in response to the rotation of the first roller 551. Similarly to the first roller 551, a lateral side of the second roller 552 is covered by the covering portion 59.

In the wire 60, a part on the proximal end side is wound around the wire winding portion 53, and a part on the distal end side passes through an opening 51o of the housing 51 and is exposed to the outside. The wire 60 is sandwiched between the first roller 551 and the second roller 552 at a position between the wire winding portion 53 and the opening 51o. The wire 60 is surrounded by the valve member 56 between the wire extruding portion 55 and the opening 51o (Fig. 5). The valve member 56 is disposed between the wire extruding portion 55 and the opening 51o to restrict the drug solution from moving into the accommodation portion 50.

The housing 51, the handle portion 52, the wire winding portion 53, the gear 54, and the wire extruding portion 55 can be made of a resin material, e.g. a polyurethane, a polypropylene, a hard polyvinyl chloride, or the like. The housing 51, the handle portion 52, the wire winding portion 53, the gear 54, and the wire extruding portion 55 may be made of the same material, or may be partially made of different materials. The valve member 56 and the covering portion 59 of the wire extruding portion 55 can be made of e.g. an elastic body such as a silicon rubber and an urethane rubber.

With such a configuration, the accommodation portion 50 allows the wire 60 to advance and retract in the lumen 10L of the catheter 10. Specifically, as illustrated in Fig. 3, when the handle portion 52 of the accommodation portion 50 is rotated in the first direction D1, the rotative force applied to the handle portion 52 is transmitted to the first gear 541 and the wire winding portion 53 through the rotation shaft O1. Thereby, the wire winding portion 53 rotates in the first direction D1, and the wire 60 having a length corresponding to the rotation quantity of the handle portion 52 is fed toward the lumen 10L (the wire 60 advances in the lumen 10L). In addition, when the handle portion 52 of the accommodation portion 50 is rotated in the second direction D2, similarly, the wire winding portion 53 rotates in the second direction D2, and the wire 60 having the length corresponding to the rotation quantity of the handle portion 52 is wound from the lumen 10L (the wire 60 retracts in the lumen 10L).

The rotation of the first gear 541 in association with the rotation of the handle portion 52 is transmitted to the third gear 543 through the second gear 542. Thereby, the first roller 551 and the second roller 552 rotate, and the wire 60 sandwiched between the first roller 551 and the second roller 552 is extruded toward the lumen 10L. Herein, as illustrated in Fig. 4, a ratio between the reference circle diameter L31 of the third gear 543 and the outer diameter L32 of the first roller 551 (L31/L32) is lower than a ratio between the reference circle diameter L11 of the first gear 541 and the outer diameter L12 of the wire winding portion 53 (L11/L12). Thus, in response to the same rotation quantity of the handle portion 52, the length of the wire 60 to be extruded by the first roller 551 and the second roller 552 is longer than the length of the wire 60 to be fed by the wire winding portion 53. In other words, in a state of sandwiching the wire 60, the first roller 551 and the second roller 552 push the wire 60 toward the lumen 10L while interposing some wheel slip.

Furthermore, since the lateral sides (the faces sandwiching the wire 60) of the first roller 551 and the second roller 552 are covered by the covering portion 59 made of the elastic body, the first roller 551 and the second roller 552 can sandwich the wire 60 and push the wire 60 toward the lumen 10L by a frictional force.

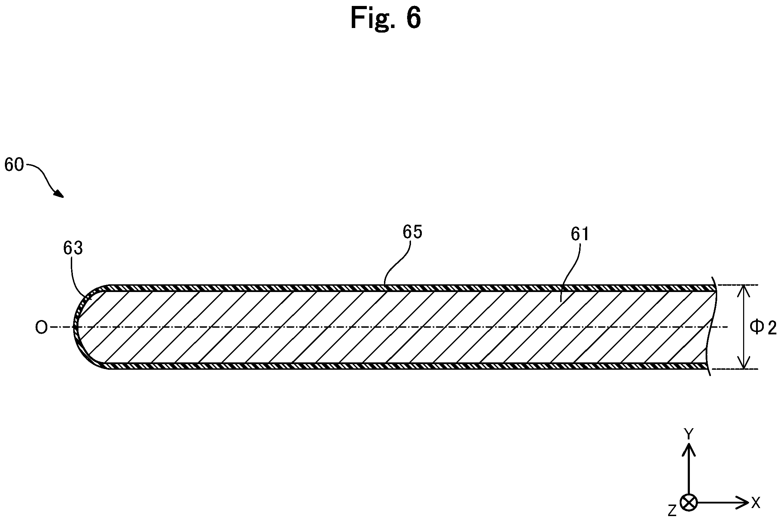

Fig. 6 is an explanatory diagram illustrating a configuration of the wire 60. The wire 60 includes a core shaft 61 and a covering portion 65. The core shaft 61 is a long member extending along the axis line O and having a substantially constant outer diameter from the distal end to the proximal end. The transverse sectional shape of the core shaft 61 is almost circular. The covering portion 65 is a coating layer applied to the surface of the core shaft 61. Similarly to the core shaft 61, the transverse sectional shape of the wire 60 is almost circular. Preferably, the core shaft 61 has antithrombogenicity, flexibility, and biocompatibility, and can be made of e.g. a metal material of a stainless steel such as SUS304, an NiTi alloy, or the like. The covering portion 65 can be made of a PTFE (polytetrafluoroethylene), a nylon, and an urethane resin. The wire 60 according to the first embodiment has a substantially constant outer diameter Φ2 from the distal end to the proximal end. The outer diameter Φ2 of the wire 60 is 80% or higher of the inner diameter Φ1 of the lumen 10L of the catheter 10 (Fig. 2).

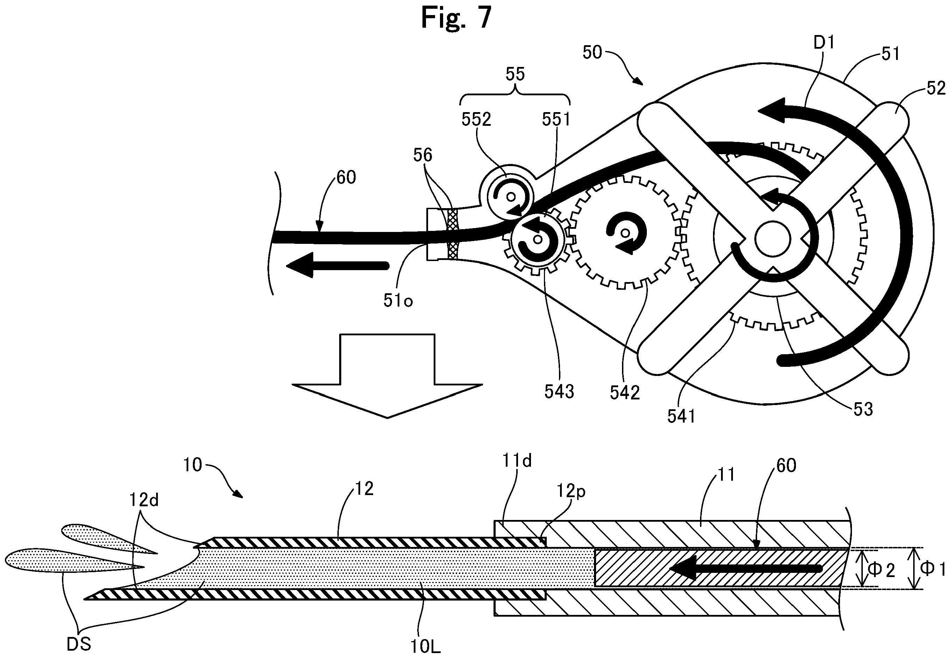

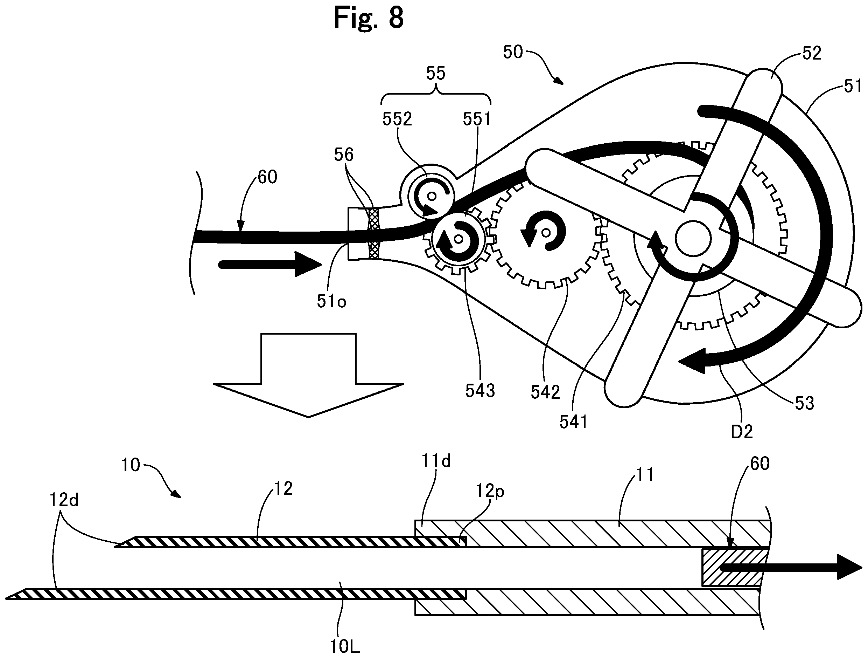

Fig. 7 is a diagram for explaining advancement of the wire 60 and discharge of the drug solution. Fig. 8 is a diagram for explaining retraction of the wire 60. In Fig. 7 and Fig. 8, the accommodation portion 50 is illustrated on the upper part, and a part of the catheter 10 on the distal end side is illustrated on the lower part. A method for using the drug solution injection device 1 will be explained with reference to Fig. 1, Fig. 7, and Fig. 8.

When using the drug solution injection device 1 by means of an endoscope, an operator inserts the catheter 10 into the endoscope to move the needle portion 12 of the catheter 10 to a target position (position to which the drug solution is administered) in a patient's body. Subsequently, the operator attaches the accommodation portion 50 to the wire insertion port 31o of the catheter 10 (Fig. 1). At this time, while the distal end portion of the wire 60 extruding from the opening 51o of the accommodation portion 50 is inserted into the lumen 10L of the catheter 10, the accommodation portion 50 is attached to the wire insertion port 31o. Then, the operator feeds the drug solution from the drug solution feed port 33o of the catheter 10. For example, a syringe containing a drug solution is attached to the proximal end portion of the second extension portion 33, and the drug solution is fed from the syringe. The drug solution fed from the drug solution feed port 33o through the second extension portion 33 is charged into the lumen 10L of the catheter 10. Subsequently, the operator punctures the target position in the patient's body with the needle portion 12.

When using the drug solution injection device 1 without using the endoscope, the operator attaches the accommodation portion 50 to the catheter 10 and inserts the catheter 10 into a living body lumen of the patient while the drug solution is previously charged into the lumen 10L of the catheter 10. Then, the operator moves the needle portion 12 of the catheter 10 to the target position in the patient's body and punctures the target position with the needle portion 12.

After puncturing the target position with the needle portion 12, the operator rotates the handle portion 52 of the accommodation portion 50 in the first direction D1, as illustrated in the upper part of Fig. 7. As a result, in association with the rotation of the handle portion 52, the first gear 541 and the wire winding portion 53 rotate in the first direction D1, and the wire 60 is fed into the lumen 10L of the catheter 10 (i.e. the wire 60 advances). Then, as illustrated in the lower part of Fig. 7, the drug solution DS charged in the lumen 10L of the catheter 10 is extruded by the advanced wire 60, and the drug solution DS in an amount corresponding to a volume of the advanced wire 60 is discharged from the distal end of the needle portion 12. That means, the drug solution DS in an amount corresponding to the rotation quantity of the handle portion 52 is discharged from the distal end of the needle portion 12. In association with the rotation of the first gear 541, the second gear 542 rotates in a direction opposite to the first direction D1, the third gear 543 and the first roller 551 rotate in the first direction D1, and the second roller 552 rotates in a direction opposite to the first direction D1. Thereby, the wire 60 is extruded into the lumen 10L of the catheter 10.

After injecting the drug solution DS into the target position in the patient's body, the operator rotates the handle portion 52 of the accommodation portion 50 in the second direction D2, as illustrated in the upper part of Fig. 8. As a result, in association with the rotation of the handle portion 52, the first gear 541 and the wire winding portion 53 rotate in the second direction D2, and the wire 60 is wound from the lumen 10L of the catheter 10 (i.e. the wire 60 retracts). In association with the rotation of the first gear 541, the second gear 542 rotates in a direction opposite to the second direction D2, the third gear 543 and the first roller 551 rotate in the second direction D2, and the second roller 552 rotates in a direction opposite to the second direction D2.

For example, a case that the drug solution DS in a larger amount than a capacity of the lumen 10L of the catheter 10 is administered to a patient is assumed. In this case, the operator continues to rotate the handle portion 52 in the second direction D2 to maximally wind the wire 60 (Fig. 1). Subsequently, the operator recharges the drug solution DS into the lumen 10L of the catheter 10 from the drug solution feed port 33o, and then only needs to repeat the operations explained in Fig. 7 and Fig. 8.

As described above, in the drug solution injection device 1 according to the first embodiment, the wire 60 that is inserted into the lumen 10L containing the drug solution DS and is advanced from the proximal end of the lumen 10L toward the distal end side (in the -X direction) so as to discharge the drug solution DS in the lumen 10L from the distal end of the needle portion 12, is included. That means, in the drug solution injection device 1 according to the first embodiment, the drug solution DS in the lumen 10L is extruded by the wire 60 so as to be discharged from the distal end of the needle portion 12 (Fig. 7). Thereby, an amount of the drug solution DS to be discharged can be the same as a volume of the wire 60, and therefore the injection dose of the drug solution DS can be precisely controlled. Thus, for example, it is also possible to cope with a case that a trace amount of drug solution DS is injected into each of multiple positions in the body. In the drug solution injection device 1 according to the first embodiment, the drug solution DS in the lumen 10L can be thoroughly used by extruding the drug solution DS in the lumen 10L by the wire 60. Thus, waste of the drug solution DS can be reduced.

In the drug solution injection device 1 according to the first embodiment, the outer diameter Φ2 of the wire 60 is substantially constant from the distal end to the proximal end (Fig. 6). Thus, the amount of the drug solution DS to be discharged from the distal end of the needle portion 12 can be easily comprehended depending on the length of the wire 60 advanced in the lumen 10L.

Furthermore, the drug solution injection device 1 according to the first embodiment includes the accommodation portion 50 connected to the proximal end portion of the catheter 10, accommodating a part of the wire 60 on the proximal end side, and allowing the wire 60 to advance and retract in the lumen 10L (Fig. 1, Fig. 5). Thereby, the wire 60 can be advanced and retracted without direct contact between an operator and the wire 60. The accommodation portion 50 feeds the wire 60 to the lumen 10L to advance the wire 60, and winds the wire 60 from the lumen 10L to retract the wire 60. Thus, the wire 60 can be reliably advanced and retracted. In addition, since the wire 60 is wound for accommodation, the accommodation portion 50 can be downsized.

Furthermore, in the drug solution injection device 1 according to the first embodiment, the accommodation portion 50 includes the handle portion 52 connected to the wire winding portion 53, and the wire winding portion 53 feeds and winds the wire 60 in a length corresponding to the rotation quantity of the handle portion 52 (Fig. 3). Thereby, the operator can easily feed and wind the wire 60 by rotating the handle portion 52. In addition, the accommodation portion 50 allows the drug solution DS in an amount corresponding to the rotation quantity of the handle portion 52 to be discharged from the distal end of the needle portion 12 (Fig. 7). Thus, the amount of the drug solution DS to be discharged from the distal end of the needle portion 12 can be easily controlled depending on the rotation quantity of the handle portion 52. As a result, the injection dose of the drug solution DS can be precisely controlled and waste of the drug solution DS can be reduced.

Furthermore, in the drug solution injection device 1 according to the first embodiment, the accommodation portion 50 includes the wire extruding portion 55 (first roller 551, second roller 552) that extrudes the wire 60 fed from the wire winding portion 53, toward the lumen 10L (Fig. 3). Thereby, the wire 60 can be reliably pushed and advanced into the lumen 10L. Specifically, in response to the same rotation quantity of the handle portion 52, the length of the wire 60 to be extruded by the wire extruding portion 55 is longer than the length of the wire 60 to be fed by the wire winding portion 53 (Fig. 3, Fig. 4). Thereby, the wire 60 can be reliably pushed and advanced into the lumen 10L.

Furthermore, in the drug solution injection device 1 according to the first embodiment, the accommodation portion 50 includes the valve member 56 that restricts the drug solution DS in the lumen 10L from moving into the accommodation portion 50. Thus, when the drug solution DS is charged into the catheter 10, the drug solution DS in the lumen 10L can be prevented from entering the accommodation portion 50 (specifically, the inside of the housing 51), and the inside of the accommodation portion 50 can be maintained in an airtight state.

Furthermore, in the drug solution injection device 1 according to the first embodiment, each transverse sectional shape of the lumen 10L of the catheter 10 and the wire 60 is almost circular, and the outer diameter Φ2 of the wire 60 is 80% or larger of the inner diameter Φ1 of the lumen 10L (Fig. 2, Fig. 5, Fig. 6). Thus, the drug solution DS in the lumen 10L can be extruded by the wire 60 while maintaining slidability of the wire 60 in the lumen 10L. Since the wire 60 according to the first embodiment includes the covering portion 65, the drug solution DS can be prevented from remaining on the surface of the wire 60.

Furthermore, in the drug solution injection device 1 according to the first embodiment, the catheter 10 further includes the connector 30 (Fig. 1). Thereby, convenience for inserting the wire 60 into the catheter 10 can be improved. The connector 30 is configured such that the drug solution feed port 33o is disposed on the distal end side with respect to the wire insertion port 31o (Fig. 1: Positions P1 and P2). Thereby, the drug solution DS can be fed from the drug solution feed port 33o without inhibition of the feed due to the wire 60, while the distal end portion of the wire 60 is inserted into the wire insertion port 31o. In addition, the drug solution feed port 33o of the connector 30 is disposed on the distal end side with respect to the distal end of the wire 60 maximally wound around the wire winding portion 53 (Fig. 1: positions P1 and P3). Thus, the drug solution DS can be fed from the drug solution feed port 33o without inhibition of the feed due to the wire 60 when the wire 60 is maximally wound around the accommodation portion 50 by rotating the handle portion 52.

Furthermore, in the drug solution injection device 1 according to the first embodiment, the connector 30 is configured such that the wire insertion port 31o is formed on the proximal end portion of the first extension portion 31, and the drug solution feed port 33o is formed on the distal end portion of the second extension portion 33 extending in a direction different from the first extension portion 31 (Fig. 1). Thereby, the accommodation portion 50 and the syringe can be attached to the connector while the accommodation portion 50 attached to the wire insertion port 31o and the syringe attached to the drug solution feed port 33o do not interfere with each other (do not disturb each other). That means, in the drug solution injection device 1 according to the first embodiment, insertion of the wire 60 into the wire insertion port 31o and feed of the drug solution DS from the drug solution feed port 33o can be smoothly carried out.

### <Second Embodiment>

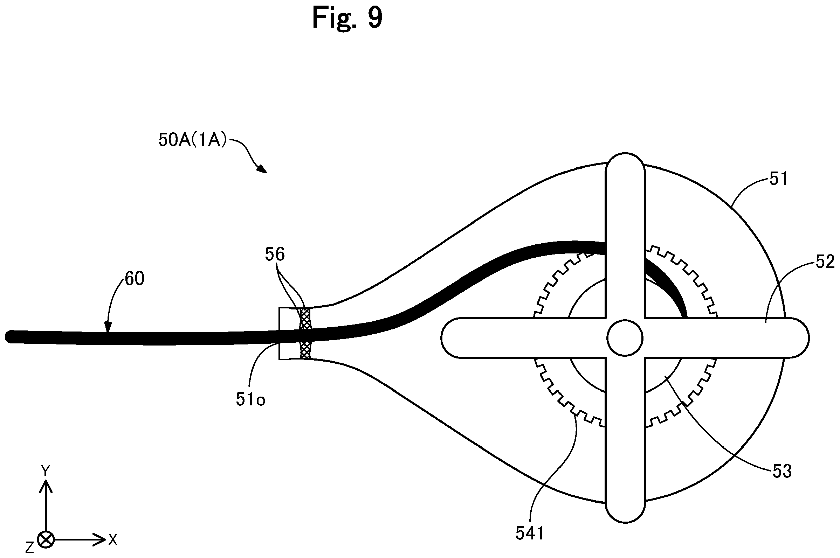

Fig. 9 is an explanatory diagram illustrating a configuration of an accommodation portion 50A according to the second embodiment. A drug solution injection device 1A according to the second embodiment includes the accommodation portion 50A in place of the accommodation portion 50 explained in the first embodiment. The accommodation portion 50A does not include the wire extruding portion 55 (first roller 551 and second roller 552), the second gear 542, and the third gear 543 that have been explained in the first embodiment. In this way, the configuration of the accommodation portion 50A can be variously changed, and the wire extruding portion 55 and the second and third gears 542 and 543 for transmitting the rotative force to the wire extruding portion 55 may be omitted. Also in this accommodation portion 50A according to the second embodiment, the wire winding portion 53 can advance the wire 60 by feeding the wire 60 into the lumen 10L and retract the wire 60 by winding the wire 60 from the lumen 10L. That means, the same effect as in the first embodiment can also be exhibited by the drug solution injection device 1A using the accommodation portion 50A according to the second embodiment.

### <Third Embodiment>

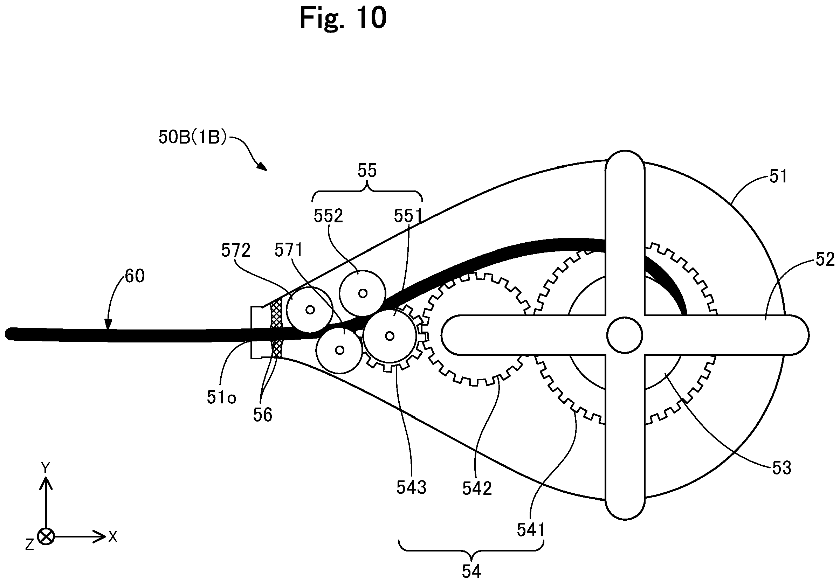

Fig. 10 is an explanatory diagram illustrating a configuration of an accommodation portion 50B according to the third embodiment. A drug solution injection device 1B according to the third embodiment includes the accommodation portion 50B in place of the accommodation portion 50 explained in the first embodiment. The accommodation portion 50B further include a third roller 571 and a fourth roller 572 in addition to the configuration explained in the first embodiment. Both the third roller 571 and the fourth roller 572 are almost cylindrical or almost columnar members. Similarly to the first roller 551 according to the first embodiment, both the lateral sides of the third roller 571 and the fourth roller 572 are covered by the covering portion 59. The third roller 571 and the fourth roller 572 are disposed between the wire extruding portion 55 and the valve member 56 (opening 51o) to sandwich the wire 60 on the distal end side with respect to the wire extruding portion 55. The third roller 571 and the fourth roller 572 rotate in response to the rotation of the wire extruding portion 55.

In this way, the configuration of the accommodation portion 50B can be variously modified, and may further include configurations not explained in the first embodiment e.g. like the third roller 571 and the fourth roller 572. The same effect as in the first embodiment can also be exhibited by the drug solution injection device 1B using the accommodation portion 50B according to the third embodiment. Also in the accommodation portion 50B according to the third embodiment, the wire 60 can be extruded toward the lumen 10L with a stronger force by the third roller 571 and the fourth roller 572.

### <Fourth Embodiment>

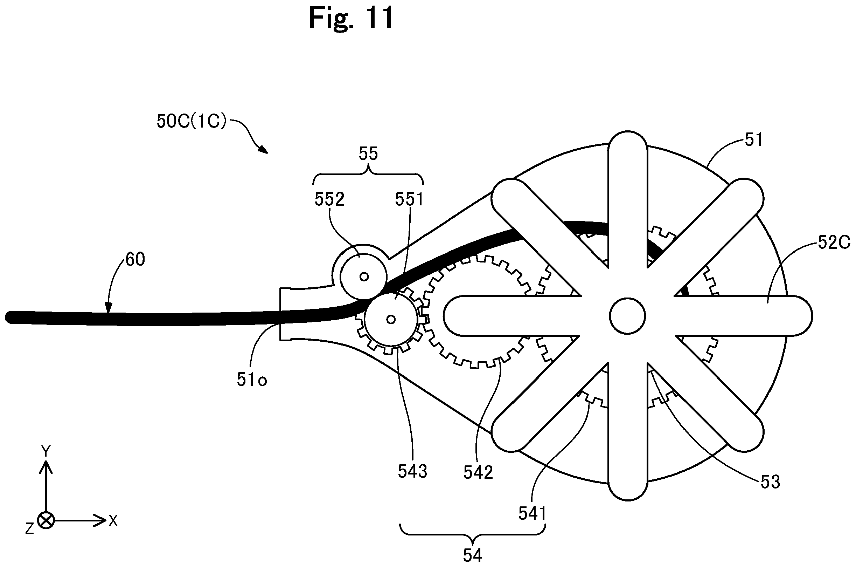

Fig. 11 is an explanatory diagram illustrating a configuration of an accommodation portion 50C according to the fourth embodiment. A drug solution injection device 1C according to the fourth embodiment includes the accommodation portion 50C in place of the accommodation portion 50 explained in the first embodiment. The accommodation portion 50C includes a handle portion 52C in place of the handle portion 52 and does not include the valve member 56 explained in the first embodiment. The handle of the handle portion 52C has a shape different from that in the first embodiment. In this way, the configuration of the accommodation portion 50C can be variously changed, and a shape of at least a part of a constituent, e.g. the handle portion 52C, as well as the housing 51, the gear 54, and the wire extruding portion 55, may be changed. For example, similarly to the valve member 56, a part of the configuration explained in the first embodiment may be omitted. The same effect as in the first embodiment can also be exhibited by the drug solution injection device 1C using the accommodation portion 50C according to the fourth embodiment.

### <Fifth Embodiment>

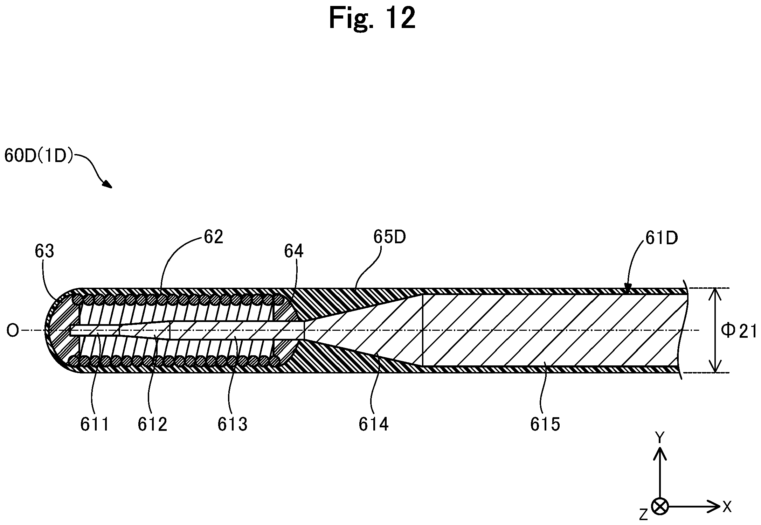

Fig. 12 is an explanatory diagram illustrating a configuration of a wire 60D according to the fifth embodiment. A drug solution injection device 1D according to the fifth embodiment includes the wire 60D in place of the wire 60 explained in the first embodiment. The wire 60D includes a core shaft 61D, a coil body 62, a distal joint part 63, a proximal joint part 64, and a covering portion 65D.

The core shaft 61D includes a first small-diameter portion 611, a first tapered portion 612, a second small-diameter portion 613, a second tapered portion 614, and a large-diameter portion 615. The first small-diameter portion 611 refers to a part where an outer diameter of the core shaft 61D is smallest, and is disposed on the distal end of the core shaft 61D. The first tapered portion 612 refers to a part having an outer diameter gradually increasing from the distal end side to the proximal end side, and is disposed between the first small-diameter portion 611 and the second small-diameter portion 613. The second small-diameter portion 613 refers to a part having a slightly larger diameter than of the first small-diameter portion 611, and is disposed between the first tapered portion 612 and the second tapered portion 614. The second tapered portion 614 refers to a part having an outer diameter gradually increasing from the distal end side to the proximal end side, and is disposed between the second small-diameter portion 613 and the large-diameter portion 615. The large-diameter portion 615 refers to a part where an outer diameter of the core shaft 61D is largest, and is disposed on the proximal end side of the core shaft 61D.

The coil body 62 is formed by spirally winding a wire around the small-diameter portion (specifically, the first small-diameter portion 611, the first tapered portion 612, and the second small-diameter portion 613) of the core shaft 61D. The coil body 62 may be a single-thread coil formed by winding one wire in a single-thread form, a multi-thread coil formed by winding a plurality of wires in a multi-thread form, a single-thread strand coil formed by winding a strand obtained by twisting a plurality of wires together in a single-thread form, or a multi-thread strand coil formed by using a plurality of strands obtained by twisting a plurality of wires together and winding each strand in a multi-thread form.

The distal joint part 63 is disposed on the distal end portion of the wire 60D and integrally holds the distal end portion of the first small-diameter portion 611 and the distal end portion of the coil body 62. The proximal joint part 64 integrally holds the proximal end portion of the second small-diameter portion 613 and the proximal end portion of the coil body 62. The covering portion 65D is a resin layer that covers the outsides of the core shaft 61D, the coil body 62, the distal joint part 63, and the proximal joint part 64. The covering portion 65D covers the respective parts so as to have such a thickness that the outer diameter of the wire 60D is an outer diameter Φ21 substantially constant from the distal end to the proximal end.

The core shaft 61D can be made of the same material as for the core shaft 61 in the first embodiment. The coil body 62 can be made of a stainless alloy such as SUS304 and SUS316, a superelastic alloy such as an NiTi alloy, a piano wire, a radiolucent alloy such as a nickel-chromium alloy and a cobalt alloy, a radiopaque alloy such as gold, platinum, tungsten, and an alloy containing any of these elements (e.g. a platinum-nickel alloy), or another known material. The distal joint part 63 and the proximal joint part 64 can be made of any joining agent, e.g. a metal solder such as silver solder, gold solder, zinc, Sn-Ag alloy, and Au-Sn alloy, or an adhesive such as an epoxy adhesive. The covering portion 65D can be made of the same material as for the covering portion 65 in the first embodiment.

In this way, the configuration of the wire 60D can be variously changed, and may be configured such that a small-diameter portion (e.g. the first small-diameter portion 611, the first tapered portion 612, and the second small-diameter portion 613) is formed on the distal end side of the core shaft 61D, and the small-diameter portion is covered with the coil body 62. The same effect as in the first embodiment can also be exhibited by the drug solution injection device 1D using the wire 60D according to the fifth embodiment. Furthermore, since use of the wire 60D according to the fifth embodiment can flexibilize the distal end portion of the wire 60D, the safety of the drug solution injection device 1D can be improved.

### <Sixth Embodiment>

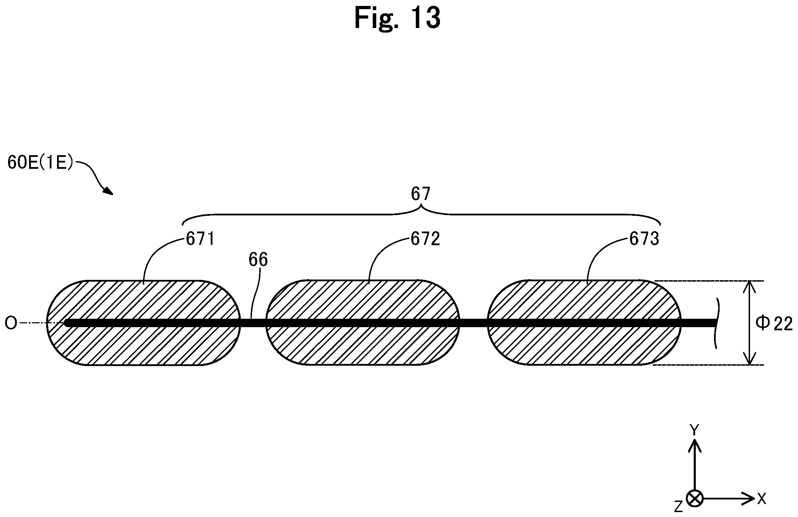

Fig. 13 is an explanatory diagram illustrating a configuration of a wire 60E according to the sixth embodiment. A drug solution injection device 1E according to the sixth embodiment includes the wire 60E in place of the wire 60 explained in the first embodiment. The wire 60E includes a core shaft 66 and a plurality of thick-walled portions 671 to 673. The core shaft 66 is a long and solid member extending along the axis line O.

Each of the thick-walled portions 671 to 673 is a member having an outer diameter larger than the outer diameter of the core shaft 66, and covers a part of the surface of the core shaft 66. Each of the thick-walled portions 671 to 673 has an almost circular transverse section, and has a substantially identical outer diameter Φ22. The thick-walled portions 671 to 673 are joined to the core shaft 66 at a distance from each other. For the joining, e.g. any joining agent such as an epoxy adhesive can be used. Although three thick-walled portions 671 to 673 are illustrated in Fig. 13, any number of thick-walled portions 671 to 673 may be formed on the wire 60E. A length in the axis line O direction and a shape of the thick-walled portions 671 to 673 can be arbitrarily determined. The distance between the adjacent thick-walled portions (in other words, the length between the separated thick-walled portions 671 to 673) can also be arbitrarily determined. The thick-walled portions 671 to 673 may be composed of a coil body formed by spirally winding a wire around the core shaft 66, and a resin body for covering the surface of the coil body.

In this way, the configuration of the wire 60E can be variously modified, and the wire 60E may be configured so as to include a plurality of thick-walled portions 671 to 673. The same effect as in the first embodiment can also be exhibited by the drug solution injection device 1E using this wire 60E according to the sixth embodiment. Furthermore, use of the wire 60E according to the sixth embodiment facilitates comprehension of an amount of the drug solution DS to be discharged from the needle portion 12 in association with the advancement of the one thick-walled portion 671 toward the lumen 10L. Thus, use of the wire 60E according to the sixth embodiment facilitates control of the amount of the drug solution DS to be discharged from the needle portion 12 depending on the number of the thick-walled portions 671 to 673 to be advanced toward the lumen 10L.

### <Seventh Embodiment>

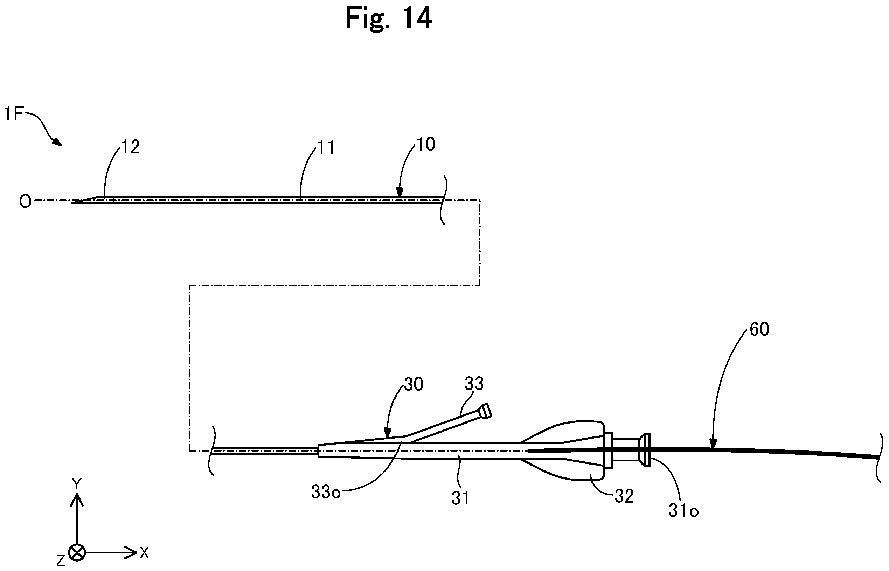

Fig. 14 is an explanatory diagram illustrating a configuration of a drug solution injection device 1F according to the seventh embodiment. The drug solution injection device 1F according to the seventh embodiment does not include the accommodation portion 50 explained in the first embodiment. In the drug solution injection device 1F according to the seventh embodiment, an operator grasps the wire 60 inserted from the wire insertion port 31o of the connector 30 by a hand (or using a grasping tool such as forceps) to advance and retract the wire 60. As described above, the configuration of the drug solution injection device 1F can be variously modified, and, for example, at least a part of the configuration explained in the first embodiment, such as the accommodation portion 50 may be omitted. This drug solution injection device 1F according to the seventh embodiment also makes it possible to discharge the drug solution DS in an amount corresponding to the length of the advanced wire 60 from the distal end of the needle portion 12. Thereby, the drug solution injection device 1F according to the seventh embodiment makes it possible to precisely control the injection dose of the drug solution DS, and reduce waste of the drug solution DS.

### <Eighth Embodiment>

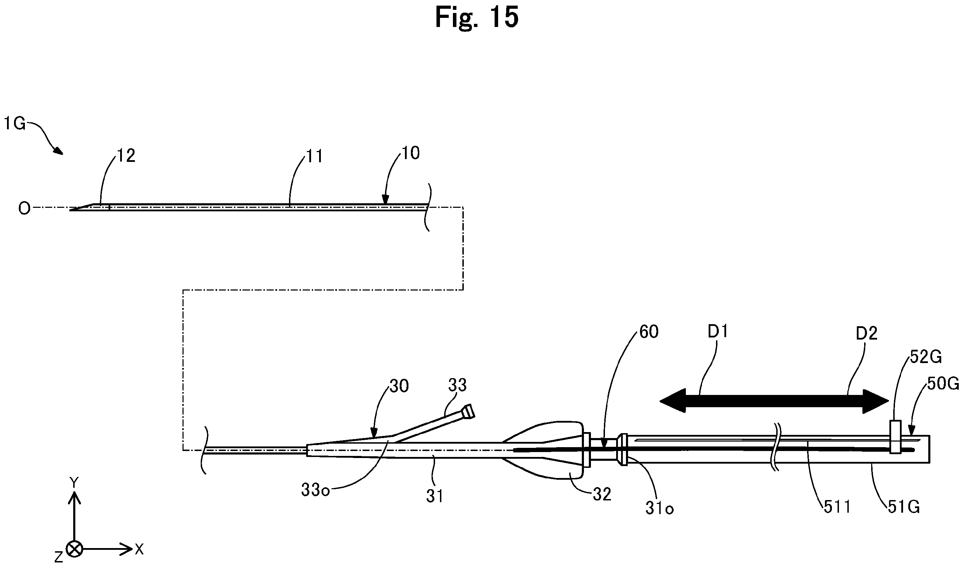

Fig. 15 is an explanatory diagram illustrating a configuration of a drug solution injection device 1G according to the eighth embodiment. The drug solution injection device 1G according to the eighth embodiment includes an accommodation portion 50G in place of the accommodation portion 50 explained in the first embodiment. The accommodation portion 50G includes a housing 51G and a handle portion 52G. The housing 51G is an almost rectangular case extending in the direction of the axis line O. A linear groove 511 that communicates between the inside and the outside of the housing 51G is formed on one side of the housing 51G. The handle portion 52G is arranged such that one end is positioned outside the housing 51G and the other end is positioned inside the housing 51G. The other end of the handle portion 52G (the end portion positioned inside the housing 51G) grasps the proximal end portion of the wire 60.

This accommodation portion 50G allows the operator to slide the handle portion 52G toward the distal end side (in other words, in the first direction D1) to advance the wire 60 into the lumen 10L. In addition, the operator can slide the handle portion 52G toward the proximal end side (in other words, in the second direction D2) to retract the wire 60 from the inside of the lumen 10L.

Thus, the configuration of the accommodation portion 50G can be variously changed, and, for example, the accommodation portion 50G may be configured such that the wire winding portion 53 and the gear 54 are not installed and the wire 60 can be advanced and retracted without winding the wire 60. Also this drug solution injection device 1G according to the eighth embodiment includes the accommodation portion 50G connected to the proximal end portion of the catheter 10, accommodating a part of the wire 60 on the proximal end side, and allowing the wire 60 to advance and retract in the lumen 10L (Fig. 15). Thereby, the wire 60 can be advanced and retracted without direct contact between an operator and the wire 60. The accommodation portion 50G makes it possible to discharge the drug solution DS in an amount corresponding to the length of the advanced wire 60 from the distal end of the needle portion 12. Thereby, the injection dose of the drug solution DS can be precisely controlled and waste of the drug solution DS can be reduced.

### <Modification Examples of the Embodiments>

The disclosed embodiments are not limited to the above embodiments, and may be implemented in various modes without departing from the gist of the disclosed embodiments. For example, the following modifications can also be applied.

### [Modification Example 1]

In the first to eighth embodiments, some examples of the configurations of the drug solution injection devices 1 and 1A to 1G have been described. However, the configuration of the drug solution injection device can be variously modified. For example, the drug solution injection device need not include any accommodation portion. For example, the drug solution injection device may include other constituents not explained in the above embodiments. As other constituents, for example, a syringe for injecting the drug solution, a controller for controlling each part of the drug solution injection device, or the like can be used.

### [Modification Example 2]

In the first to eighth embodiments, an example of the configuration of the catheter 10 has been described. However, the configuration of the catheter can be variously modified. For example, the catheter may be configured as a multi-lumen catheter including another lumen different from the lumen for the drug solution. For example, a valve member for restricting reverse flow of the drug solution (movement toward the wire insertion port side or the drug solution feed port side) may be disposed in the lumen of the catheter. For example, the catheter may include a mesh member for positioning the needle portion in a living body lumen. For example, the catheter may include a balloon member for intercepting current of body fluids (e.g. blood) flowing through the living body lumen and positioning the needle portion.

For example, in the axis line O direction, the drug solution feed port and wire insertion port of the connector may be disposed at the almost same position. For example, in the axial line O direction, the drug solution feed port of the connector may be disposed on the proximal end side with respect to the wire insertion port. For example, in the axis line O direction, the drug solution feed port of the connector and the distal end of the wire maximally wound around the accommodation portion may be positioned at the almost same position. For example, in the axis line O direction, the drug solution feed port of the connector may be disposed on the proximal end side with respect to the distal end of the wire maximally wound around the accommodation portion.

For example, the connector need not include the second extension portion having the drug solution feed port. In this case, the operator may operate the wire by attaching the syringe to the wire insertion port, then charging the drug solution into the syringe, then detaching the syringe, and attaching the accommodation portion to the same wire insertion port.

### [Modification Example 3]

In the first to eighth embodiments, some examples of the configurations of the accommodation portions 50, 50A to 50C, and 50G are described. However, the configuration of the accommodation portion can be variously modified. For example, the accommodation portion may include an electric handle portion that is driven by an electric motor or the like in place of a manually-rotatable handle portion. In this case, the accommodation portion may further include an input portion for inputting a feed amount of the drug solution (or a rotation quantity of the electric handle portion). The input portion can be achieved in any form such as a touch panel or operation buttons. For example, the accommodation portion may include a fixation member for fixing the accommodation portion to the wire insertion port of the connector.

For example, the reference circle diameter L11 of the first gear, the reference circle diameter L31 of the third gear, the outer diameter L12 of the wire winding portion, and the outer diameter L32 of the first roller can be arbitrarily determined. The ratio between the reference circle diameter L31 of the third gear and the outer diameter L32 of the first roller (L31/L32) is not necessarily smaller than the ratio between the reference circle diameter L11 of the first gear and the outer diameter L12 of the wire winding portion (L11/L12). For example, the first gear and the third gear need not have the inner teeth and outer teeth. In this case, the first gear and the wire winding portion may be joined to each other by any joining agent such as an epoxy adhesive. Similarly, the third gear and the first roller may be joined to each other by any joining agent such as an epoxy adhesive. For example, in the accommodation portion, the rotative force of the handle portion may be transmitted to the wire winding portion and the wire extruding portion by using a means other than the gears. As the means other than the gears, for example, a pulley and a belt, a magnetic force, or the like can be used.

### [Modification Example 4]

In the first to eighth embodiments, some examples of the configurations of the wires 60, 60D, and 60E have been described. However, the configuration of the wire can be variously modified. For example, the outer diameter of the wire is not necessarily substantially constant from the distal end to the proximal end. In this case, the wire may be configured such that a part on the distal end side is formed so as to have a relatively narrow diameter, and a part on the proximal end side is formed so as to have a relatively thick diameter. For example, the outer diameter of the wire may be less than 80% of the inner diameter of the lumen. For example, the transverse sectional shape of the wire is not necessarily almost circular.

### [Modification Example 5]

The configurations of the drug solution injection devices according to the first to eighth embodiments and the configurations of the drug solution injection device, the catheter, the accommodation portion, and the wire according to Modification Examples 1 to 4 may be combined as appropriate. For example, the wire explained in any of the fifth and sixth embodiments may be used in the drug solution injection device explained in the first, seventh, and eighth embodiments. For example, the accommodation portion explained in any of the second, third, and fourth embodiments may be used in the drug solution injection device having the configuration explained in the first embodiment.

Although the aspects have been explained above based on the embodiments and the modification examples, the embodiments of the aforementioned aspects are made for facilitating understanding of the aspects, and does not limit the aspects. The aspects can be modified and improved without departing from the spirit of the aspects and the scope of the claims, and equivalent aspects are included in the aspects. Further, unless the technical features are described as essential in the present specification, the technical features may be omitted as appropriate.

### DESCRIPTION OF REFERENCE NUMERALS

- 1, 1A to 1G: Drug solution injection device
- 10: Catheter
- 10L: Lumen
- 11: Hollow shaft
- 12: Needle portion
- 30: Connector
- 31: First extension portion
- 31o: Wire insertion port
- 32: Blade portion
- 33: Second extension portion
- 33o: Drug solution feed port
- 50, 50A to 50C, 50G: Accommodation portion
- 51, 51G: Housing
- 52, 52C, 52G: Handle portion
- 53: Wire winding portion
- 54: Gear
- 55: Wire extruding portion
- 56: Valve member
- 59: Covering portion
- 60, 60D, 60E: Wire
- 61, 61D: Core shaft
- 62: Coil body
- 63: Distal joint part
- 64: Proximal joint part
- 65, 65D: Covering portion
- 66: Core shaft
- 511: Groove
- 541: First gear
- 542: Second gear
- 543: Third gear
- 551: First roller
- 552: Second roller
- 571: Third roller
- 572: Fourth roller
- 611: First small-diameter portion
- 612: First tapered portion
- 613: Second small-diameter portion
- 614: Second tapered portion
- 615: Large-diameter portion
- 671, 672, 673: Thick-walled portion

## Claims

1. A drug solution injection device comprising:
a catheter that has a hollow shaft, a hollow needle portion disposed on a distal end of the hollow shaft, and a lumen formed inside each of the hollow shaft and the needle portion and containing a drug solution; and
a wire that is inserted into the lumen and is advanced from a proximal end of the lumen toward a distal end side so as to discharge the drug solution in the lumen from a distal end of the needle portion.

2. The drug solution injection device according to claim 1, wherein
an outer diameter of the wire is substantially constant from a distal end to a proximal end.

3. The drug solution injection device according to claim 1 or 2, further comprising:
an accommodation portion that is connected to a proximal end portion of the catheter, accommodates a part of the wire on the proximal end side, and allows the wire to advance and retract in the lumen.

4. The drug solution injection device according to claim 3, wherein
the accommodation portion advances the wire in the lumen, so that the drug solution in an amount corresponding to a length of the advanced wire is discharged from the distal end of the needle portion.

5. The drug solution injection device according to claim 3 or 4, wherein
the accommodation portion feeds the wire toward the lumen to advance the wire, and winds the wire from the lumen to retract the wire.

6. The drug solution injection device according to claim 5, wherein
the accommodation portion comprises:
a wire winding portion around which a part of the wire on a proximal end side is wound; and
a handle portion connected to the wire winding portion, and
the wire winding portion feeds and winds the wire in a length corresponding to a rotation quantity of the handle portion.

7. The drug solution injection device according to claim 6, wherein
the accommodation portion further comprises:
a wire extruding portion that extrudes the wire fed from the wire winding portion, toward the lumen, and
the wire extruding portion includes first and second rollers that rotate in response to the rotation of the handle portion, and extrudes the wire while the wire is sandwiched between the first and second rollers.

8. The drug solution injection device according to claim 7, wherein
in response to the same rotation quantity of the handle portion,
the length of the wire to be extruded by the wire extruding portion is longer than the length of the wire to be fed by the wire winding portion.

9. The drug solution injection device according to claim 8, wherein
the accommodation portion further comprises:
a valve member that is disposed between the wire extruding portion and the proximal end portion of the catheter to restrict the drug solution in the lumen from moving into the accommodation portion.

10. The drug solution injection device according to any one of claims 6 to 9, wherein
the accommodation portion allows the drug solution in an amount corresponding to the rotation quantity of the handle portion to be discharged from the distal end of the needle portion.

11. The drug solution injection device according to any one of claims 1 to 10, wherein
the catheter further comprises:
a connector having a drug solution feed port for feeding the drug solution to the lumen and a wire insertion port for inserting the wire into the lumen, connected to a proximal end portion of the hollow shaft, and in communication with the lumen, and
the drug solution feed port is disposed on a distal end side with respect to the wire insertion port.

12. The drug solution injection device according to any one of claims 6 to 10, wherein
the catheter further comprises:
the connector having a drug solution feed port for feeding the drug solution to the lumen and a wire insertion port for inserting the wire into the lumen, connected to the proximal end portion of the hollow shaft, and in communication with the lumen, and
the drug solution feed port is disposed on a distal end side with respect to the distal end of the wire maximally wound around the wire winding portion by the rotation of the handle portion.

13. The drug solution injection device according to claim 11 or 12, wherein
the connector further comprises:
a first extension portion extending in a longitudinal direction of the catheter; and
a second extension portion extending from a lateral side of the first extension portion in a direction different from the first extension portion on a distal end side of the first extension portion, and
the drug solution feed port is formed on a distal end portion of the second extension portion, and the wire insertion port is formed on a proximal end portion of the first extension portion.

14. The drug solution injection device according to any one of claims 1 to 13, wherein
each transverse sectional shape of the lumen and the wire is almost circular, and
an outer diameter of the wire is 80% or larger of an inner diameter of the lumen.
